# EUROPEAN PATENT APPLICATION

(11) **EP 2 017 247 A1**
(43) Date of publication of application: **21.01.2009**
(21) Application number: 07743043.7
(22) Date of filing: 09.05.2007
(51) Int. Cl.: C07C 5/31, C07C 13/615, C07B 61/00, C07C 7/10, C07C 13/547

(54) **PROCESS FOR PRODUCING ADAMANTANE**

(30) Priority: 10.05.2006 JP 2006131754
(71) Applicant: Idemitsu Kosan Co., Ltd., Chiyoda-ku Tokyo 100-8321 (JP)
(72) Inventor: SAITO, Masao, Ichihara-shi Chiba 299-0193 (JP); KOJIMA, Akio, Ichihara-shi Chiba 299-0193 (JP); KUSABA, Toshiaki, Ichihara-shi Chiba 299-0193 (JP); MASE, Jun, Ichihara-shi Chiba 299-0193 (JP)
(74) Representative: Vossius & Partner
(86) International application number: PCT/JP2007/059608
(87) International publication number: WO 2007/129738

(57) **Abstract**

Provided is a method for production of adamantane which enables producing a high-purity adamantane at low cost and with high efficiency by using effectively trimethylenenorbornane contained in a heavy raffinate heavy and having conventionally been no use other than fuels and the like, and which enables alleviating poisoning of a catalyst used in isomerization and corrosion of devices, whereby an industrially advantageous adamantane can be produced. In addition, the process for producing adamantane including an isomerization process of isomerizing trimethylenenorbornane contained in a raffinate obtained from a platfinate is **characterized by** including a water-washing removal process of removing sulfolane in a trimethylenenorbornane concentrate to be supplied in the isomerization process by water-washing.

## Description

### Technical Field

The present invention relates to a method for production of adamantane.

### Background Art

Adamantane has a structure in which four cyclohexane rings are condensed into a cage-shape, thereby being a stable and highly symmetrical compound. It has been known that adamantane having such an adamantane skeleton exhibits peculiar functions, thereby being useful for lubricants, agricultural and medical raw materials, highly functional industrial materials, and the like.
As a method for production of adamantane, there is generally adopted a method, in which trimethylenenorbornane obtained by hydrogenating dicyclopentadiene is isomerized.
In the isomerization, aluminum chloride is generally used as a catalyst.
However, the yield of adamantane when being produced by using aluminum chloride as a catalyst is about 50 mass% (for example, Patent Documents 1 and 2). In this case, the catalyst must be used in a large amount, and further, cannot be reused because the catalyst forms a complex with a heavy component during the reaction.
Therefore, when the above-mentioned method is used, a large amount of waste aluminum is produced, and the disposal of the waste aluminum causes the problems of environmental pollution. Further, because the produced adamantane is colored in a case of using aluminum chloride, it is necessary to conduct a decoloring process through recrystallization or by using an active carbon. Thus, there is a problem in that post treatment becomes inevitably troublesome.

There is also known a solid catalyst which contains an active metal such as platinum, rhenium, nickel, or cobalt carried on a cation-exchanged zeolite by an impregnation method (for example, Patent Document 3).
A method for production of adamantane using above-mentioned catalyst in which the active metal such as platinum, rhenium, nickel, or cobalt is carried on the cation-exchanged zeolite by the impregnation method can solve the above-mentioned problems caused in the case where the aluminum chloride catalyst is used.
However, the above-mentioned conventional methods using the aluminum chloride catalyst and the solid catalyst have a problem in that the production of adamantane unavoidably requires high cost because trimethylenenorbornane used as a starting material is obtained by hydrogenating expensive dicyclopentadiene.

In order to solve those problems, there is suggested a method for production of adamantane through the isomerization etc., using trimethylenenorbornane as a raw material by focusing attention on the fact that the trimethylenenorbornane is contained in a raffinate obtained from a platfinate (for example, Patent Document 4).
According to the production method described above, a high-purity adamantane can be produced at low cost and with high efficiency, thereby being capable of solving the above-mentioned problems. However, the raffinate used as a raw material contains sulfolane used for extracting an aromatic fraction in the platfinate, resulting in problems such as poisoning of a catalyst and corrosion of devices used in the isomerization. In addition, in a case where a heavy raffinate heavy, which is a fraction obtained by removing a light gas from the raffinate, is used as a raw material, similar problems have occurred.
Patent Document 1: JP 50-71663 A
Patent Document 2: JP 2000-143556 A
Patent Document 3: JP 52-2909 B
Patent Document 4: WO 05/058779

### Disclosure of the Invention

### Problems to be solved by the Invention

In such circumstances, an obj ect of the present invention is to provide a method for production of an industrially advantageous adamantane, which enables producing a high-purity adamantane at low cost and with high efficiency and lowering of poisoning of a catalyst or corrosion of devices used in the isomerization.

### Means for solving the Problems

The inventors of the present invention extensively studied to achieve the above-mentioned object. As a result, the inventors have found that the poisoning of the catalyst and the corrosion of devices used in the isomerization can be lowered as a result of removing sulfolane contained in a heavy raffinate heavy obtained from a platfinate by washing with water. The present invention has been completed based on this finding.

That is, the present invention provides a method for production of adamantane as described below:
1. a method for production of adamantane including an isomerization process of isomerizing trimethylenenorbornane contained in a heavy raffinate heavy obtained from a platfinate, characterized by including a water-washing removal process of removing sulfolane in a trimethylenenorbornane concentrate to be supplied in the isomerization process by water-washing;
2. a method for production of adamantane according to the item 1, in which a sulfolane concentration of the trimethylenenorbornane concentrate is 10 ppmor less after the water-washing removal process; and
3. a method for production of adamantane according to the item 1, in which a sulfolane concentration of the trimethylenenorbornane concentrate is 0.01 to 10 ppm after the water-washing removal process.

### Effects of the Invention

According to the method for production of adamantane of the present invention, the high-purity adamantane can be produced at low cost and with high efficiency and the poisoning of the catalyst and the corrosion of devices used in the isomerization can be lowered, whereby the industrially advantageous adamantane can be produced.

### Best Mode for carrying out the Invention

### [Heavy raffinate heavy: starting material]

In the method for production of adamantane according to the present invention, trimethylenenorbornane contained in a heavy raffinate heavy obtained from a platfinate is used as a starting material.
The term "platfinate" used herein is a fraction obtained by hydrogenating olefins contained in a pyrolysis gasoline obtained as a result of steam cracking of naphtha and removing a light gas therefrom. In addition, the term "raffinate" herein is a residual fraction (containing a naphthene having 5 to 11 carbon atoms and an aromatic having 6 to 11 carbon atoms in addition to trimethylenenorbornane and methyl trimethylenenorbornane) obtained by extracting an aromatic fraction contained in the above-mentioned platfinate with sulfolane or the like. The heavy raffinate heavy is a fraction (containing a naphthene having 6 to 10 carbon atoms and an aromatic having 6 to 9 carbon atoms in addition to trimethylenenorbornane and methyl trimethylenenorbornane) obtained by further removing a light gas such as a light raffinate or Ipsol M after the raffinate was washed with water for the purpose of removing sulfolane. Accordingly, dicyclopentadiene in the pyrolysis gasoline is converted to trimethylenenorbornane by hydrogenation and, trimethylenenorbornane is contained in the platfinate, the raffinate, and also in the heavy raffinate heavy. That is, the present invention effectively use trimethylenenorbornane contained in the heavy raffinate heavy and having conventionally been no use other than fuels and the like.

The method for production of adamantane of the present invention is a method for production of adamantane using trimethylenenorbornane contained in the heavy raffinate heavy as a raw material, and the method must include a water-washing removal process (B), describedbelow, of removing sulfolane by water-washing. An example of a preferred production method includes, in the stated order, a concentration process of trimethylenenorbornane (A), a water-washing removal process (B), an isomerization process (C), a concentration process of adamantane (D), a crystallization process (E), a solid-liquid separation process (F), a washing process (G), and a drying process (H). Note that, the adamantane (hereinafter, sometimes referred to as adamantanes) obtained by the production method of the present invention is a hydrocarbon compound having an adamantane structure, and alkyl substituents of the adamantane having a low alkyl group such as a methyl group or an ethyl group are exemplified other than the adamantane.
Hereinafter, each process is described.

[(A) Concentration process of trimethylenenorbornane] The heavy raffinate heavy can be used as it is, but a trimethylenenorbornane is preferably concentrated for use from the viewpoint of production efficiency. The concentration process is for obtaining a trimethylenenorbornane concentrate from the heavy raffinate heavy.
In the concentration process, methylcyclohexane, dimethylcyclohexane, and the like are removed with a multistage distillation column equipped with stages or a filling distillation column equipped with a filler such as Raschig ring, whereby the concentration is completed with one distillation column. The concentration of trimethylenenorbornane in the outlet of the distillation column is generally 3 to 90 mass%, or preferably 50 to 80 mass%. When the concentration of trimethylenenorbornane is in above-mentioned range, the production amount of the adamantane per hour in the isomerization process (C) is improved, and the size of the reactor can be reduced.
A light fraction obtained from the column top of the distillation column can be converted again to a useful petrochemical raw material such as ethylene or propylene by using part or all of the light fraction as a raw material of an ethylene cracking furnace.
In addition, as a concentration process for trimethylenenorbornane contained in the heavy raffinate heavy, an atmospheric distillation or a reduced-pressure distillation is preferred. Upon the concentration, by reducing the content of the trimethylenenorbornane having an alkyl group and contained in the heavy raffinate heavy, the production amount of an alkyl adamantane (such as methyl adamantane) in the isomerization solution can be reduced. As a result, a high-purity adamantane can be obtained.

### [(B) Water-washing removal process]

The water-washing removal process (B) is a process of removing the trimethylenenorbornane concentrate obtained in the concentration process of trimethylenenorbornane (A) by water-washing, and is an essential process in the production method of the present invention.
Because the aromatic fraction in the platfinate is extracted with sulfolane or the like as described above, the sulfolane remains in the raffinate. Before the raffinate is obtained, the sulfolane in the raffinate is generally removed by water-washing once for the purpose of preventing contamination of the sulfolane into the raffinate. However, in the heavy raffinate heavy obtained thereafter, about 100 to 300 ppm of sulfolane is generally remained. When the raffinate containing the sulfolane is concentrated in the concentration process of trimethylenenorbornane (A), the amount of sulfolane in the trimethylenenorbornane concentrate tends to increase to generally about 300 to 900 ppm, resulting in poisoning of a catalyst used in the isomerization process or corrosion of devices used in (C) to (H) processes described below. Therefore, the water-washing removal process (B) is essentially required for the production method of the present invention for the purpose of reducing the sulfolane content in the trimethylenenorbornane concentrate.

The sulfolane content in the trimethylenenorbornane concentrate after the water-washing removal process is preferably 10 ppmor less, more preferably 1 ppm or less, and still more preferably 0.01 ppm or more. In addition, the combination of the upper limit and lower limit is preferred. When the content is in the range, the poisoning of the catalyst process and the corrosion of devices used in the isomerization as described above can be prevented.
The water-washing removal process in the water-washing removal process is not particularly limited as long as the sulfolane content in the trimethylenenorbornane concentrate can be reduced to above-mentioned range. For example, an alternative derivation type extract method using a spray column equipped with a porous plate or a filling column filled with Raschig ring or the like is preferably exemplified. In addition, the amount of water to be supplied in the water-washing removal process with respect to the trimethylenenorbornane amount is, depending on a desired sulfolane concentration, generally 0.5 to 10 kg/(kilograms of trimethylenenorbornane concentrate) and preferably 1 to 5 kg/(kilograms of trimethylenenorbornane concentrate).

### [(C) Isomerization process]

The isomerization process (C) is a process of producing adamantanes by isomerizing the trimethylenenorbornane obtained in the water-washing removal process (B) with a batchmode or a continuous mode.
As the catalyst used for isomerization in this process, there may be mentioned acid catalysts such as an aluminum chloride catalyst, and solid catalysts. In consideration of the troublesome post treatment after isomerization, the solid catalyst is preferably used. As the solid catalyst, there may be mentioned a solid acid catalyst carrying a metal.
As the solid acid, there may be mentioned zeolites (A-type, L-type, X-type, Y-type, ZSM-5, and the like), sulfated zirconia, silica-alumina, alumina,and hetero polyacids. Of those, a zeolite, particularly, the Y-type zeolite is preferred.
As the metal, there may be mentioned metals belonging to the group 8 to the group 10 of the periodic table and rhenium. Of those, ruthenium, rhodium, palladium, iridium, platinum, and rhenium are preferred. Platinum is more preferred.

The solid catalyst may be prepared by known methods. For example, a process for preparing a solid catalyst in which a metal is carried on a zeolite includes causing a zeolite to carry at least one kind of metal by an ion exchange process or an impregnation process. In the case of the ion exchange process, for example, the catalyst may be prepared by bringing an aqueous solution of a metal salt or an aqueous solution of a metal complex of above-mentioned metals into contact with the zeolite to subjecting an ion-exchange of a cation (such as H⁺ and NH₄⁺) in the zeolite, followed by drying and calcination. In addition, in the case of the impregnation process, for example, after the zeolite is mixed with and an aqueous solution of a metal salt or an aqueous solution of a metal complex, the mixture is evaporated and dried to obtain a solid, whereby the catalyst may be prepared by impregnating above-mentioned metal to be carried on the zeolite.
The shape of the solid catalyst is not specifically limited. A catalyst of any desired shape such as a powder or a granule may be used.

Regarding the reaction conditions for isomerization of trimethylenenorbornane using the solid catalyst, the reaction temperature is generally 150 to 500°C or preferably 200 to 400°C, and the pressure is normal pressure or increased pressure.
Although not particularly limited, the style of the reactor may be a circulation mode reactor, a batch mode reactor, or the like. When the reaction is performed in the batch mode, the reaction time is about 1 to 50 hours.
Further, the reaction is preferably carried out in the presence of hydrogen from the viewpoint of improving the yield of the adamantane.
In the isomerization of the isomerization process, the reaction can be performed in the coexistent of a monocyclic saturated hydrocarbon compound, an aromatic compound, water and/or an alcohol in the presence of the solid catalyst. Specific examples of the compound to be coexisted are described in WO 05/058779.

### [Concentration process of adamantane (D)]

The concentration process (D) is a process of subjecting the product solution of isomerization obtained in the isomerization process (C) to concentration treatment with a flush column alone, a distillation column alone, or a plural flush columns or distillation columns in combination according to a batch mode or a continuous mode to remove a solvent or a light by-product (impurity), and concentrating to such a concentration that the product solution can be crystallized efficiently in the subsequent crystallization process (E). The concentration process is performed preferably when the concentration of the adamantanes in the product solution of isomerization is less than 10 mass%.
In the concentration process (D), in general, an unreacted light gas such as hydrogen is removed by using a flash column, and the concentration is completed by using one distillation column. In addition, the adamantane concentration at the final stage of the concentration process is generally 10 to 50 mass%, or preferably 20 to 40 mass%. When the concentration is within the range, because the recovery efficiency of the adamantane in the crystallization process (E) is not decreased, and the slurry viscosity upon the crystallization is not increased, the operation becomes easy.

Part or all of the light fraction obtained from overhead of the distillation column is used as a solvent in the isomerization process (C) or the like, and hence additional use amount of the reaction solvent can be suppressed to the minimum, and a raw material and a reaction intermediate fraction contained in the light fraction or the adamantanes can be used by recycle. In addition, part or all of the overhead liquid is used as a recrystallization solvent in the subsequent process, that is, crystallization process (E), and hence an additional amount of the recrystallization solvent can be suppressed to the minimum. The recycle amount for the isomerization process (C) or the use amount of the recrystallization solvent is appropriately adjusted by the adamantane concentration in the fraction obtained from the overhead in the distillation column or the concentrations of other impurities, or a flow balance in the process. As a result, a product with an intended purity can be obtained efficiently.

### [(E) Crystallization process]

The crystallization process (E) is a process of crystallizing the adamantanes from the product solution of the isomerization obtained in the isomerization process (C) or the concentrate obtained in above-mentioned concentration process of the adamantane (D) according to a batch mode or a continuous mode.
General cooling crystallization or evaporation crystallization, or a combination of them can be employed as a crystallization operation. The operation temperature in the crystallization operation depends on the concentration of the product solution of the isomerization or the adamantane concentration of the concentrate. In the case of continuous crystallization, the operation temperature is ordinarily about -20 to 50°C, or preferably 0 to 30°C. When the temperature condition is in above-mentioned range, a large amount of energy is not consumed for cooling, whereby preferable recovery efficiency of the adamantanes can be obtained. In the case of the batch mode as well, due to the same reason, the final temperature is preferably -20 to 50°C and more preferably adjusted to 0 to 30°C. In addition, in any other crystallization processes, it is advantageous to set the final temperature in the crystallization process to the temperature at which the solubility of the adamantanes is about 0.5 to 25 mass%, or preferably about 5 to 15 mass % because of the same reason.
When an impurity causing a problem in terms of quality is taken in the concentrate through one crystallization, recrystallization may be performed immediately after the crystallization. Alternatively, after the solid-liquid separation process (F) and the washing process (G) as post-processing processes, the recrystallization, the solid-liquid separation process, and the washing process may be repeated multiple times. In addition, in the case where an adamantane having a purity more than the intended purity is obtained through one crystallization process, the obtained crystal may be solved in a general organic solvent, and be crystallized. Note that an organic solvent having low solubility to adamantane is not preferred. Examples of the crystallization solvent, that is, the unsuitable organic solvent include alcohols, ketones, and carboxylic acids.

### (F) Solid-Liquid Separation Process

The solid-liquid separation process (F) is a process of separating the adamantane crystals precipitated in the crystallization process (E) and the solvent according to a batch mode or a continuous mode.
As the solid-liquid separation operation, a general method using a filter cloth, a sintered metal, or the like may be used. In this case, part of the separated mother liquid is generally exhausted outside the system in order to suppress the concentration of impurities. Then, by recycling part or all of the remainder in the concentration process or crystallization process, loss of the adamantanes can be suppressed to the minimum. Alternatively, by using part or all of the remainder as part of the solvent or raw material in the isomerization process or the like, a novel solvent is not needed, or a raw material and a reaction intermediate fraction contained in the light fraction, or the adamantanes can be recycled and used.
The solid-liquid separation is performed in such a degree that the liquid content in the separated crystal cake is preferably about 50 mass% or less, and more preferably 5 to 30 mass%.
The exhaust amount of the mother liquid to outside the system or the recycle amount of the mother liquid is appropriately adjusted by the concentration of the adamantanes contained in the mother liquid or the concentration of the other impurities, or flow balance in the process, whereby a product with an intended purity can be obtained efficiently.

### (G) Washing Process

The washing process is a process of washing and removing the solvent that has not been sufficiently removed in the solid-liquid separation process (F) by using a washing solvent.
As the washing solvent, almost all organic solvents can be used, but polar solvents such as alcohols, ketones, carboxylic acids each having low solubility are preferred in order not to decrease the recovering efficiency of the adamantanes. In addition, in the case where the washed adamantanes are directly treated in the subsequent process, that is, the drying process (H), a solvent having a low boiling point, which is easily dried, is preferred. In general, a solvent having a boiling point of 150°C or lower is suitable. Examples of the solvent include methanol, ethanol, propanol, isopropanol, acetone, methyl ethyl ketone, diethyl ketone, acetic acid, and carbon tetrachloride.
The washing operation temperature ordinarily is in the range of room temperature to a temperature equal to or lower than the boiling point of the washing solvent, or preferably -20 to 50°C.

### (H) Drying Process

The drying process is a process of subjecting the washed adamantane crystals obtained in above-mentioned washing process (G) to drying treatment.
A general drying machine that has been industrially used, such as a reduced-pressure-type drying machine or a heating-type drying machine, can be used in the drying treatment. In addition, a drying method may follow one of a continuous mode and a batch mode.
Because the object of the drying treatment lies in the removal of the washing solvent, the operating conditions for the treatment vary depending on the kind of a solvent to be used in the washing process. In ordinary cases, the pressure is equal to or lower than normal pressure, or preferably 5 to 101 kPa, and the temperature is equal to or lower than the boiling point of the washing solvent, or preferably 20 to 60°C.
Thus, no troublesome operation such as waste liquid disposal is needed, and high-purity adamantanes can be efficiently produced while the loss thereof is minimized.

### Examples

Next, the present invention is described in detail by way of examples, but the present invention is not limited thereto. In the following examples and comparative example, evaluations of the production method of the present invention were performed as described below.

### (1) Poisoning evaluation of isomerization catalyst

A long lifetime test was performed for poisoning evaluation for an isomerization catalyst. A fixed bed circulation mode reactor was filled with a 0.9% Pt/REY zeolite catalyst and operated continuously for 800 hours at a reaction temperature of 325°C, a reaction pressure of 5 MPa, and WHSV of 0.5 h⁻¹. A product solution of the isomerization was analyzed by gas chromatography, whereby a product amount of adamantanes in the reaction solution of the isomerization was obtained. A ratio at which trimethylenenorbornane in a trimethylenenorbornane concentrate at the initial isomerization process was converted into an adamantane (trimethylenenorbornane conversion ratio) was calculated by using the product amount. Then, an effect of poisoning due to sulfolane was evaluated with a decrease (difference) of the conversion ratio from initial operation.

### (2) Corrosion evaluation of device

A simulation pipe formed of carbon steel was provided at the outlet of the fixed bed circulation reactor. After the poisoning evaluation for the isomerization catalyst, the pipe was cut, and then corrosion degree inside the pipe was evaluated by observation of the surface and with a penetrant test (PT test) in accordance with JIS Z2343-1982.

### Example 1

### (1) Catalyst Preparation

1,275 g of a Y-type zeolite having sodium ions in its cation sites (hereinafter referred to as NaY) were added and suspended in 7 kg of pure water with stirring, and the suspension was heated to 60°C.
While the mixture was stirred, 8 kg of an aqueous solution of mixed rare earth chlorides (mixed chlorides containing 49 mass% of Ce, 24 mass% of La, 20 mass% of Nd, 5 mass% of Pr, and 2 mass% of Sm) [containing 890 g in terms of RE₂O₃ (total of CeO₂ + La₂O₃ +Nd₂O₃+Pr₂O₃+Sm₂O₃)] were added to the mixture, followed by stirring for 2 hours.
The solids were then collected by filtration and washed with 15 kg of pure water.
After that, the washed product was dried and thereafter calcined at 650°C for 3 hours (primary exchange with mixed rare earth).
340 g of the thus calcined powder were suspended in 2 kg of warm water at 60°C, and hydrochloric acid was added to the mixture with sitrring until the pH of the mixture reached 5.01.
The thus obtained slurry was mixed with 2 kg of the aqueous solution of mixed rare earth chlorides described above [130.6 g in terms of RE₂O₃ (the same as above)], and the mixture was stirred at 60°C for 2 hours.
The solids were then collected by filtration and washed with 4 kg of pure water (secondary exchange with mixed rare earth).
340 g of the thus obtained powder were suspended again in 2 kg of pure water, and 340 g of 1. 81 mass% aqueous tetrammine-platinum chloride solution were added to the mixture. The mixture was stirred at 30°C for 2 hours.
The resultant was filtered and washed, and then dried overnight at 110°C, whereby an uncalcined solid catalyst of a mixed rare earth-containing Y-type zeolite (REY zeolite) carrying 1.0 mass% of platinum was obtained.

### (2) water-washing removal process to isomerization process

In a stainless steel reaction tube, 20 g of the catalyst obtained in the section (1) above were filled and calcined at 300°C for 3 hours in the stream of air.
After substitution with nitrogen, hydrogen reduction was carried out at 300°C for 2 hours under normal pressure in the stream of hydrogen.
After that, the heavy raffinate heavy was distilled under normal pressure, whereby a trimethylenenorbornane concentrate in which the trimethylenenorbornane concentration was reduced to 80 mass% and the methyl trimethylenenorbornane concentration was reduced to 0.79 mass% was obtained. The sulfolane concentration in the concentrate was 900 ppm. Next, the obtained trimethylenenorbornane concentrate was washed with water by applying water at a ratio of 1 kg/(kilograms of trimethylenenorbornane), and the sulfolane was removed by water-washing until the sulfolane concentration reached to 1 ppm. Supply of the concentrate containing sulfolane at low concentration and hydrogen was started, and the concentrate was isomerized continuously under the condition of 325°C, 5MPa, WHSV of 0.5 h⁻¹ (TMN standard), and molar ratio of hydrogen to TMN of 2.

### (3) Post Isomerization Process

600 g of the thus obtained isomerization solution (adamantane (ADM) concentration: 4.3 mass%) were concentrated by distillation under normal pressure until the adamantane concentration reached 27 mass%.
The concentrate was heated to 120°C with stirring to dissolve deposited adamantane and then cooled to 10°C with stirring to crystallize the adamantane, whereby a slurry containing deposited adamantane was obtained.
The slurry was then filtered through a 70-µm glass filter, and a crude adamantane crystal was obtained.
Isopropyl alcohol was added to the crude adamantane crystal on the 70-µm filter, and then the mixture was filtered by vacuum, whereby the adamantane crystal was washed by substitution.
The thus obtained adamantane crystal was dried with air to evaporate the isopropyl alcohol, whereby 40 g of the adamantane crystal was obtained.
The adamantane crystal was analyzed by gas chromatography to reveal that the purity of the adamantane crystal was 97.3 mass% and that 1.1 mass% of unreacted trimethylenenorbornane and 1. 6 mass% of by-products were included as impurities.

### Example 2

Adamantane was produced by the same process as in Example 1 except that the sulfolane in the concentrate was removed by water-washing until the sulfolane concentration reached 10 ppm in the water-washing removal of the sulfolane conducted in Example 1. Table 1 shows evaluation results.

### Example 3

Adamantane was produced by the same process as in Example 1 except that the sulfolane in the concentrate was removed by water-washing until the sulfolane concentration reached 100 ppm in the water-washing removal of the sulfolane conducted in Example 1. Table 1 shows evaluation results.

### Comparative Example 1

Adamantane was produced by the same method as in Example 1 except that no water-washing removal of the sulfolane conducted in Example 1 was conducted. Table 1 shows evaluation results.

Example 1 and 2 showed excellent results in the poisoning evaluation for the isomerization catalyst, and the corrosion evaluation of devices. In addition, Example 3 showed a good result that the conversion ratio was reduced by 20% in the poisoning evaluation for the isomerization catalyst, which was not problematic for practical use, and showed excellent results in the corrosion evaluation. In Comparative Example 1, in which the water-washing removal of the sulfolane was not conducted, there were remarkable poisoning of the isomerization catalyst and corrosion of devices.

[Table 1]

**Table 1**

| | Example 1 | Example 2 | Example 3 | Comparative Example 1 |
|---|---|---|---|---|
| Poisoning evaluation for isomerization catalyst | No change | No change | Reduction by 20% | Reduction by 75% |
| Corrosion evaluation for devices (PT test) for devices (PT test) | No corrosion | No corrosion | No corrosion | Corrosion |

### Industrial Applicability

According to the method for production of adamantane of the present invention, the high-purity adamantane can be produced at low cost and with high efficiency by using effectively trimethylenenorbornane contained in the heavy raffinate heavy and having conventionally been no use other than fuels and the like, and poisoning of a catalyst and corrosion of devices used in the isomerization can be lowered, whereby the industrially advantageous adamantane can be produced.

## Claims

1. A method for production of adamantane including an isomerization process of isomerizing trimethylenenorbornane contained in a heavy raffinate heavy obtained from a platfinate, **characterized by** comprising a water-washing removal process of removing sulfolane in a trimethylenenorbornane concentrate to be supplied in the isomerization process by water-washing.

2. A method for production of adamantane according to claim 1, wherein a sulfolane concentration of the trimethylenenorbornane concentrate is 10 ppm or less after the water-washing removal process.

3. A method for production of adamantane according to claim 1, wherein a sulfolane concentration of the trimethylenenorbornane concentrate is 0.01 to 10 ppmafter the water-washing removal process.
